# EUROPEAN PATENT SPECIFICATION

(11) **EP 4 650 460 B1**
(45) Date of publication and mention of the grant of the patent: **13.05.2026**
(21) Application number: 24382521.3
(22) Date of filing: 14.05.2024
(51) Int. Cl.: C12Q 1/6886, G01N 33/574

(54) **METHODS FOR CLASSIFYING, DETERMINING A TREATMENT AND PREDICTING THE PROGNOSIS OF A GASTRIC ADENOCARCINOMA, AND THE KIT FOR USE THEREOF**
VERFAHREN ZUR KLASSIFIZIERUNG, BESTIMMUNG EINER BEHANDLUNG UND VORHERSAGE DER PROGNOSE EINES MAGENADENOKARZINOMS UND KIT ZUR VERWENDUNG DAVON
PROCÉDÉS DE CLASSIFICATION, DE DÉTERMINATION D'UN TRAITEMENT ET DE PRÉDICTION DU PRONOSTIC D'UN ADÉNOCARCINOME GASTRIQUE ET KIT POUR SON UTILISATION

(43) Date of publication of application: 19.11.2025
(73) Proprietor: Institut de Recerca Biomèdica de Lleida Fundació Dr Pifarré, 25198 Lleida (ES)
(72) Inventor: MONTAL ROURA, Robert, 25198 Lérida (ES)
(74) Representative: Ungria López, Javier

(56) References cited:
- HU JIA-LI ET AL: "Identification of a novel inflammatory-related gene signature to evaluate the prognosis of gastric cancer patients", vol. 16, no. 3, 15 March 2024 (2024-03-15), pages 945 - 967, XP093215083, ISSN: 1948-5204, Retrieved from the Internet <URL:https://f6publishing.blob.core.windows.net/c16cd32b-1acc-4999-9889-9acebc4d2a65/WJGO-16-945.pdf> DOI: 10.4251/wjgo.v16.i3.945
- VEAS JOEL ET AL: "PD-2 Comprehensive immunophenotyping of gastric adenocarcinoma identifies an inflamed class of tumors amenable to immunotherapies", ESMO 25TH WORLD CONGRESS ON GASTROINTESTINAL CANCER 2023, BARCELONA, SPAIN, 28 JUNE - 1 JULY 2023, 31 July 2023 (2023-07-31), pages 1 - 1, XP093215093, Retrieved from the Internet <URL:https://www.postersessiononline.eu/173580348_eu/congresos/WCGIC2023/aula/-PD_2_WCGIC2023.pdf>
- VEAS J. ET AL: "PD-2 Comprehensive immunophenotyping of gastric adenocarcinoma identifies an inflamed class of tumors amenable to immunotherapies", ANNALS OF ONCOLOGY, vol. 34, 1 June 2023 (2023-06-01), pages S1, XP093215108, ISSN: 0923-7534, Retrieved from the Internet <URL:https://www.annalsofoncology.org/action/showPdf?pii=S0923-7534(23)00171-0> DOI: 10.1016/j.annonc.2023.04.029
- WEI YUANFENG ET AL: "Inflammation-Related Genes Serve as Prognostic Biomarkers and Involve in Immunosuppressive Microenvironment to Promote Gastric Cancer Progression", FRONTIERS IN MEDICINE, vol. 9, 16 March 2022 (2022-03-16), XP093215156, ISSN: 2296-858X, DOI: 10.3389/fmed.2022.801647
- KORTEKAAS KIM E ET AL: "Primary vulvar squamous cell carcinomas with high T cell infiltration and active immune signaling are potential candidates for neoadjuvant PD-1/PD-L1 immunotherapy", vol. 9, no. 10, 1 October 2021 (2021-10-01), pages e003671, XP055866901, Retrieved from the Internet <URL:https://jitc.bmj.com/content/jitc/9/10/e003671.full.pdf?with-ds=yes> DOI: 10.1136/jitc-2021-003671
- ZHOU XIN ET AL: "Relationships of tumor differentiation and immune infiltration in gastric cancers revealed by single-cell RNA-seq analyses", CMLS CELLULAR AND MOLECULAR LIFE SCIENCES, BIRKHAUSER VERLAG, HEIDELBERG, DE, vol. 80, no. 2, 1 February 2023 (2023-02-01), XP037938640, ISSN: 1420-682X, [retrieved on 20230202], DOI: 10.1007/S00018-023-04702-1

## Description

### TECHNICAL FIELD

The invention refers to the field of medicine, specifically to the personalized medicine. More specifically, the invention refers to methods for identifying the class of gastric adenocarcinoma, as well as methods of diagnosis based on said assessment, and the method for predicting responsiveness or non-responsiveness to a treatment for patients with gastric adenocarcinoma. The invention also refers to the kit for carrying out said methods.

### BACKGROUND OF THE INVENTION

Gastric cancer (GC) is the fourth leading cause of cancer death with approximately 800,000 deaths, and more than one million of new cases per year. Although the pathogenesis of GC remains unclear, chronic gastritis is believed to participate in cellular changes, leading to malignant transformation. Adenocarcinoma is the most common histologic type of GC and accounts for approximately 85 to 90% of all cancers of the stomach. Gastric adenocarcinoma (GAC) is further classified into intestinal, and diffuse histologic subtypes and into chromosomal unstable, genome stable, microsatellite unstable and Epstein-Barr positive molecular subtypes (TCGA classification).

Despite improvements in prevention, early diagnosis, and multidisciplinary therapies, GC is usually diagnosed at advanced stages leading to poor prognosis. In advanced clinical stages, cytotoxic chemotherapy is still the cornerstone of treatment, with a median overall survival of~12 months for patients treated with conventional chemotherapy. Intratumoral and intertumoral heterogeneity are prominent features of GC that partly contribute to its poor prognosis. However, histological classification alone is insufficient to effectively stratify patients for individualized treatment and improve patients' clinical outcomes. Therefore, cutting-edge diagnostic techniques and drugs are of fundamental importance for better characterizing molecular profiles and identifying potential novel therapeutic targets for GC patients.

For patients with advanced and metastatic GC, targeted therapy represents a promising treatment option. This approach comprises the identification of molecular targets in cancer cells and the development of drugs capable of blocking or inhibiting these targets. Trastuzumab, a monoclonal antibody targeting Human Epidermal Receptor 2 (HER2), was the first approved targeted therapy for GC, and since its approval has contributed to improve the overall survival in patients with metastatic HER2-positive GC when it is added to chemotherapy. Anti-vascular endothelial growth factor (VEGF) therapy is another example of targeted therapy that, in combination with chemotherapy, has demonstrated enhanced survival rates compared to chemotherapy alone. Due to its improved efficacy outcome, monoclonal VEGFR-2-targeting antibody ramucirumab has been recommended as the standard treatment for advanced GC in the second-line setting.

In recent years, immunotherapy has showed promising outcomes, particularly for patients with advanced, recurrent, or metastatic GC. Among the many alternatives, one of the most commonly utilized is the use of Immune Checkpoint Inhibitors (ICIs), such as anti-Programmed death protein 1 (PD-1)/Programmed death-ligand 1 (PD-L1). By blocking related immune checkpoint signaling pathways, ICls can restore the anti-tumor immune responses of immune cells. The good survival outcomes obtained in clinical trials measuring the efficacy of anti-PD-1/PD-L1, validated the use of chemotherapy plus nivolumab/pembrolizumab as a standard of care option in the first-line for advanced/metastatic tumors with an overexpression of PDL-1.

Due to its significant association between its level of expression and the benefit derived of immune checkpoint inhibition in GC, PD-L1 expression has been increasingly utilized for the selection of patients most likely to respond to ICI treatment. Nevertheless, this biomarker shows several issues that need to be considered in the therapeutic decision, such as such as spatial and temporal heterogeneity, interobserver variability, immunohistochemistry assay, and influence by chemotherapy or radiation therapy. Therefore, the significance of PD-L1 as a predictive biomarker for GC still needs further study, and a real need to identify patients who will truly benefit from ICI intervention in the GC space still remains.

Despite of the beneficial therapeutic effects presented by ICls, only a limited subset of patients shows complete response to PD-1 therapy, and some of the patients who show a response eventually develop resistance to immunotherapy. While the mechanisms of acquired resistance remain to be fully elucidated, there are actionable strategies that are emerging as alternate to anti-PD-1 blockade to modulate immune cell activity to overcome resistance to existing approved immunotherapies.

Treatment with anti-Cytotoxic T-lymphocyte associated protein 4 (CTLA-4) agents has been shown to have a synergistic effect with anti-PD-1 blockade, and this combination is already being tested in advanced-phase trials for GAC. In addition, there are several other immune checkpoint targets such as LAG3, TIM-3 and TIGIT that are under clinical development and may represent future effective therapies for GAC.

One aspect of the heterogeneity of GAC resides within the tumor microenvironment, which is highly infiltrated by modified fibroblasts, known as cancer-associated fibroblasts, immune cells, gastric myofibroblasts and endothelial cells, among others. The tumor microenvironment in GC is vital in propelling cell growth, defense against host immune mechanisms and treatment resistance. Despite the limitations in the interpretation of PD-L1 expression, recent clinical trial results have been consistent to suggest that the subgroup of GC patients that do not express PD-L1 in the tumor microenvironment derive little, if any, benefit from ICI treatment.

Key genes and signaling pathways have been shown to regulate tumor behavior, and are the basis for plenty of inventions in the field of biotechnology. One example such inventions are the methods that can be utilized to diagnose cancer. WO2022046576A1 discloses a method of detecting pancreatic ductal adenocarcinoma in a sample from a subject based on evaluating increased or decreased expression of signature genes selected from CD74, CD5, CCL20 and lymphocyte function-associated antigen-1, among others. Further, the reference also discloses comparing the increased or decreased expression of said genes with the normalized gene expressions. The gene expressions are detected by utilizing microarray-based spatial transcriptomics.

Similarly, JP2021126107A discloses a method and a kit for detecting a genetic marker for predicting the responsiveness of a pancreatic cancer and biliary cancer chemotherapeutic agent by transferring peripheral blood of a subject. Further, the reference discloses evaluating increased or decreased expression of genes selected from CD74 and CD3E, among others. The reference also discloses comparing the increased or decreased expression of these genes with respect to control gene expression values; wherein the gene expressions can be measured by measuring the gene, measuring the transcript of the gene including mRNA or cDNA.

Furthermore, US20190314410A1 discloses a method of treating a malignant tumor selected from stomach cancer and tumor in small intestine, in a patient. The method comprising analyzing a tumor biopsy from the patient to characterize the tumor microenvironment. Said tumor microenvironment is characterized using gene expression profiling and the T cell density within the tumor. The gene expression profiling comprises determining the expression level of a specified panel of genes by evaluating increased or decreased expression of said genes selected from CD74, CD5, MHC, CD11A, CD3E, CCL20, lymphocyte function-associated antigen-1, among others.

Other references of the prior art that have to be taken into account are: Hu Jia-Li et al. (2024), World Journal of Gastrointestinal Oncology, vol. 16(3): 945-967; Veas Joel et al. (2023), ESMO 25th World Congress on Gastrointestinal Cancer 2023; and Kortekaas Kim et al. (2021) Journal for Immunotherapy of Cancer, 9(10).

Taken together, although the understanding of mechanisms of efficacy and resistance to ICIs in GAC has advanced, the failure to identify appropriate predictive biomarkers to implement trial-enrichment strategies has limited the success of these therapies. With the aim to provide a comprehensive characterization of the tumor microenvironment of GAC, the inventors have conducted an integrative molecular analysis of tumor infiltrating immune cells in terms of type, functional status and spatial distribution that have uncovered oncogenic programs governing specific immunophenotypes with potential sensitivity to specific immunotherapies.

### DESCRIPTION OF THE INVENTION

### General description

Based on an innovative gene signature, the present invention allows the classification of a GAC tumor into two different categories: tumors that belong to the inflamed class, and tumors that don't belong to the inflamed class. Based on differential gene expression, and for the first time, GAC tumors are able to be distinguished into categories that entail different prognosis and allow the stratification of the patient, who could benefit from a more personal treatment decision based on this new tumor classification. By identifying a gene signature, the present invention characterizes an optimal subset of genes that allow the identification of tumors that belong to each category to interrogate their clinical utility.

The invention provides methods, kits, and means of detecting such gene signature to perform an analysis of a GAC tumor tissue sample.

The present invention is firstly directed to an in vitro method for determining the class of a tumor from an individual with GAC as the one of claim 1, through the gene signature. Specifically, the method comprises:
- determining in a tumor sample from said individual the level of gene expression of a gene signature, wherein the signature comprises a first subgroup consisting of CD74, CD5, HLA-E, CD3E and ITGAL; and a second subgroup consisting of CCL20, BLM, ADORA2A, RPL23 and GLS;
wherein the tumor is classified being or not of the inflamed class.

The term "individual", as used herein, refers to all animals classified as mammals and includes, but is not restricted to, domestic and farm animals, primates and humans, e.g., human beings, cows, horses, pigs, sheep, goats, dogs, cats, or rodents. Preferably, the individual is a male or female human of any age or race.

The term "gastric adenocarcinoma" is used in the broadest sense and refers to (i) all stages and all forms of cancer arising from epithelial cells lining the stomach, including the gastroesophageal junction, and/or (ii) all stages and all forms of cancer affecting the lining of the stomach, including the gastroesophageal junction.

In the present invention, the term "sample" means biological material isolated from a subject. It is within the purview of one of skill in the art to obtain a suitable sample for determining gene expression. Suitable samples include tumor sample biopsies. Such biopsies include a resected lesion (e.g., wide-excision removal of a tumor). Samples may be processes or preserved by any means known in the art to be compatible with gene expression profiling. For example, the sample may be a formalin fixed paraffin embedded primary GAC lesion biopsy, as well as a frozen sample. Preferably, the sample is an RNA-containing sample. General methods for mRNA extraction are well known in the art and are disclosed in standard textbooks of molecular biology, including Ausubel et al. (1997) Current Protocols of Molecular Biology, John Wiley and Sons. Methods for RNA extraction from paraffin embedded tissues are disclosed, for example, in Rupp & Locker (1987) Lab Invest. 56:A67, and De Andres et al., BioTechniques 18:42044 (1995). In particular, RNA isolation can be performed using purification kit, buffer set and protease from commercial manufacturers, such as Qiagen, according to the manufacturer's instructions (QIAGEN Inc., Valencia, Calif.). For example, total RNA from cells in culture can be isolated using Qiagen RNeasy mini-columns. Numerous RNA isolation kits are commercially available and can be used in the methods of the invention.

The so-called Inflamed class GAC tumors are characterized by a high tumor immunogenicity and cytotoxicity, particularly in the tumor center when compared to the invasive margin, with enrichment of PIK3CA and ARID1A mutations and increased presence of exhausted CD8+ T cells as well as co-inhibitory receptors such are PD-1, CTLA-4, LAG3 and TIGIT. On the contrary, the GAC tumors not belonging to the inflamed class are based on the observed exclusion of T cell infiltration mediated by myeloid-derived suppressor cells (MDSC), with an overexpression of VEGFA and higher presence of TP53 mutations, resulting in a worse clinical outcome. The invention discloses that the tumor microenvironment that forms the GAC of the inflamed class was proinflammatory, leucocyte rich and characterized by higher immunoreactivity. In terms of spatial protein profiling, tumors from the inflamed class were characterized by increased expression by immune cells of the major histocompatibility complex (MHC) II molecule HLA-DR as well as T cell regulation and cytotoxicity markers such as STING, exclusively in the tumor center and not in the tumor margin.

The Weighted Voting classification with Leave-One-Out Cross-Validation algorithm from GenePattern was used to select a weighted linear combination of relevant genes obtained in the study cohort set to provide a classification scheme for new samples. The selection of classifier input features (marker genes) was accomplished by computing a T-Test. The model was tested in the leave-one-out cross-validation mode by iteratively leaving one sample out and training a model on the remaining data and testing on the left-out sample. The initial selection of 10 genes per class for the construction of the gene signature seemed likely to be large enough to be robust against noise and small enough to be applied in a clinical setting.

To better understand the tumor-immune interface of GAC, the inventors assessed a study cohort of 82 patients that allowed an integrative molecular analysis including mutational profiling by whole-exome sequencing, RNA gene-expression of 770 genes associated with immune response and multiplex protein expression at spatial resolution of 34 immuno-oncology targets at different regions and compartments (tumoral cells and immune cells). In the gene-expression analysis of the cells integrating the tumor microenvironment, the RNA was isolated from frozen samples using the Maxwell RSC Simply RNA kit. RNA was used for gene expression profiling through the nCounter23 PanCancer IO 360, a curated panel including 770 genes across key immuno-oncology pathways and processes.

Clustering is a powerful machine learning tool for detecting structures in datasets. In the medical field, clustering has been proven to be a powerful tool for discovering patterns and structure in labeled and unlabeled datasets. Unlike supervised methods, clustering is an unsupervised method that works on datasets in which there is no outcome (target) variable nor is anything known about the relationship between the observations, that is, unlabeled data. Unsupervised clustering applied to the tumor microenvironment gene-expression results (770 genes related with different features of the anti-tumoral immune response as well as the composition of the immune cells infiltrating the tumor) proposed the presence of essentially two molecular classes of GAC. The first category, including 52% of the tumors in the study cohort of 82 patients, was named as "the inflamed class" based on the enrichment of gene-expression signatures defining tumor immunogenicity and anti-tumor immune activity. The second category, including the rest 48% of the tumors of the study cohort, was named as not belonging to "the inflamed class" during the assay, based on the opposite gene-expression profile suggesting lower immunogenicity (presumably as a result of a more efficient DNA damage repair).

Taking into account gene signatures that define tumor immunogenicity, tumor sensitivity to immune attack, inhibitory immune mechanisms, anti-tumor immune activity and immune cell population, the inventors designed a gene signature for the identification of tumors of these two classes, for stratifying patients based on this new classification, and to interrogate the response to immunotherapies.

The essential gene signature comprises at least 4 genes that are selected from a first subgroup consisting of CD74, CD5, HLA-E, CD3E and ITGAL; and at least 4 genes that are selected from a second subgroup consisting of CCL20, BLM, ADORA2A, RPL23 and GLS. These ten genes are a composition of immuno-supportive and immunosuppressive genes that relate to different features of the anti-tumoral immune response as well as the composition of the immune cells infiltrating the tumor:
- Cluster of Differentiation 74 (CD74) (NM_001025159.3): The term "Cluster of Differentiation 74" or CD74, as used herein, refers to a gene that encodes for a protein that associates with MHC-II. CD74 is an important chaperone that regulates antigen presentation for the immune response. It is a cell surface receptor for Macrophage Migration Inhibitory Factor that upon activation initiates survival pathways and cell proliferation. CD74 also regulates T-cell and B-cell developments, dendritic cell (DC) motility, macrophage inflammation, and thymic selection. CD74 plays important roles in many inflammatory diseases, such as liver fibrosis, type I diabetes, systemic lupus erythematosus, and Alzheimer disease. The receptor CD74 was also recently identified as a regulator of oncogenic properties in various cancers, and was positively associated with immune infiltration in advanced solid tumor microenvironment. Experimental Therapeutics: Anti-CD74 antibody-drug conjugates.
- Cluster of differentiation 5 (CD5) (NM_001346456.2): The term "Cluster of differentiation 5" or CD5, as used herein, refers to a gene from the SRCR superfamily associated with the immune system. CD5 is a receptor that is involved in regulating T-cell proliferation. In both T-cells and B-cells, CD5 associates with their antigen-specific receptor complexes and modulates their signals both qualitatively and quantitatively. CD5 is a member of the scavenger receptor cysteine-rich superfamily that is expressed on T cells and a subset of B cells (B1a) and can regulate the T cell receptor signaling pathway. Blocking CD5 function may have therapeutic potential in treatment of cancer by enhancing cytotoxic T lymphocyte recognition and ablation of tumor cells. Experimental Therapeutics: Anti-CD5 CAR T Cells.
- HLA class I histocompatibility antigen, alpha chain E (HLA-E) (NM_005516.6): The term "HLA class I histocompatibility antigen, alpha chain E" or HLA-E, as used herein, refers to a gene that encodes the HLA-E protein that binds a restricted subset of peptides derived from signal peptides of classical MHC class I molecules, namely HLA-A, B, C, G. This allows HLA-E to assemble correctly and to be expressed on the cell surface. NK cells recognize the HLA-E + peptide complex and produces an inhibitory effect on the cytotoxic activity of the NK cell to prevent cell lysis. HLA-E has been first described as the ligand of CD94/NKG2 receptors expressed mainly by natural killer (NK) cells, thus confining its role to the regulation of NK-cell function. However, recent evidences indicate that HLA-E complexed with peptides can interact with alpha beta T-cell receptor (TCR) expressed on CD8(+) T cells. HLA-E is frequently upregulated in many cancers, suggesting that it functions as an acquired resistance mechanism after immune activation in the tumor microenvironment. Experimental Therapeutics: HLA-E targeted binding peptides.
- Cluster of differentiation 3 epsilon (CD3E) (NM_000733.4): The term " Cluster of Differentiation 3-Epsilon" or CD3-Epsilon" or CD3E, as used herein, refers to a gene that encodes for a subunit of T-cell receptor complex. The complex plays an important role in coupling antigen recognition to several intracellular signal-transduction pathways. The epsilon polypeptide plays an essential role in T-cell development. Defects in this gene cause immunodeficiency. It has been shown that we found that CD3E might serve as indicators of TME modulation in bladder cancer. Therapy targeting CD3E may have the potential as novel therapeutic strategy. Experimental Therapeutics: Anti-CD3E monoclonal antibodies.
- Integrin alpha L (ITGAL) (NM_002209.3): The term "Integrin alpha L" or ITGAL, as used herein, refers to an integrin subunit alpha L chain. Integrins are heterodimeric integral membrane proteins composed of an alpha chain and a beta chain. This I-domain containing alpha integrin combines with the beta 2 chain (ITGB2) to form the integrin lymphocyte function-associated antigen-1 (LFA-1), which is expressed on all leukocytes. LFA-1 plays a central role in leukocyte intercellular adhesion through interactions with its ligands, intercellular adhesion molecules 1 through 3, and also functions in lymphocyte costimulatory signaling. Integrin alpha L (ITGAL) is a member of the integrin family in which the abnormal expression is linked with carcinogenesis and immune regulation. Upregulated ITGAL expression was strongly connected with immunomodulators, chemokines, and infiltrating levels of CD8+, CD4+ T cell, B cell, monocyte, neutrophil, macrophage, T-cell regulatory, NK cell, and myeloid DC in stomach adenocarcinoma. Specifically, immunohistochemistry and bioinformatic analysis showed that ITGAL expression was shown to have strong relationships with various immunological marker sets including PD-1 (T-cell exhaustion marker). In conclusion, ITGAL is a prognostic biomarker for GC individuals. It might regulate tumor immune microenvironment leading to poor prognosis.
- Chemokine ligand 20 (CCL20) (NM_004591.3): The term "Chemokine ligand 20" or CCL20, as used herein, refers to an antimicrobial gene that belongs to the subfamily of small cytokine CC genes. Cytokines are a family of secreted proteins involved in immunoregulatory and inflammatory processes. The CC cytokines are proteins characterized by two adjacent cysteines. The protein encoded by this gene displays chemotactic activity for lymphocytes and can repress proliferation of myeloid progenitors. CCL20 is a chemokine that binds to its receptor CCR6, and plays an important role in immune diseases such as rheumatoid arthritis, psoriasis, and other diseases. It has been demonstrated that CCL20 also contributes to the progression of many cancers, such as liver cancer, breast cancer and GC. Importantly, CCL20 can recruit immune cells such as DCs and Treg cells, which further links CCL20 with tumor microenvironment. Experimental Therapeutics: GSK3050002, a humanized IgG1_{K} antibody with high binding affinity to human CCL20.
- Bloom syndrome protein (BLM) (NM_000057.4): The term "Bloom syndrome protein" or BLM, as used herein, refers to a Bloom syndrome-associated helicase that unwinds a variety of DNA substrates including Holliday junction, and is involved in several pathways contributing to the maintenance of genome stability. It has been found that BLM mutations are associated with increased tumor mutation burden, more immune-active tumor microenvironment and improved survival after immunotherapy. In pancreatic adenocarcinoma, highly aberrant expression of BML correlates with malignant progression and immune filtration.
- Adenosine A2A receptor (NM_000675.6): The term "Adenosine A2A receptor" or ADORA2A, as used herein, refers to a gene that encodes a member of the G protein-coupled receptor superfamily. Transmembrane signal cascade, respond to extracellular cues and activate intracellular signal transduction pathways. This protein, an adenosine receptor of A2A subtype, uses adenosine as the preferred endogenous agonist. Role in many functions, including immune system. Malfunction associated with inflammatory diseases. Adenosine signaling has emerged as a key metabolic pathway that regulates tumor immunity. Adenosine is an immunosuppressive metabolite produced at high levels within the tumor microenvironment. Adenosine signaling through the A2a receptor expressed on immune cells potently dampens immune responses in inflamed tissues. A2aR signaling on both effector and regulatory T cells triggers the increased expression of other immune checkpoint pathways, including PD-1, CTLA-4, and LAG-3. Thus, A2aR signaling may represent a master checkpoint pathway.
- Ribosomal protein L23 (NM_000978.4): The term "ribosomal protein L23" or RPL23, as used herein, refers to a gene that encodes a ribosomal protein that is a component of the 60S subunit. RPL23 has been identified as a tumor metastasis driver in HCC. HCC tissue presents significantly upregulated in HCC tissues compared to adjacent normal tissues. RPL23 depletion inhibited HCC cell proliferation, migration and invasion, and distant metastasis. RPL23 might play an important role in HCC metastasis in an MMP9-dependent manner and be a potential therapeutic target for HCC tumorigenesis and metastasis.
- Glutaminase (NM_014905.5): The term "Glutaminase" or GLS, as used herein, refers to a gene that encodes the K-type mitochondrial glutaminase. This protein is primarily expressed in the brain and kidney plays an essential role in generating energy for metabolism, synthesizing the brain neurotransmitter glutamate and maintaining acid-base balance in the kidney. Glutaminases are encoded by two different genes called GLS1 and GLS2. While GLS1 is usually upregulated in cancers, the expression of GLS2 is generally repressed in cancers. GLS1 is more likely to be tumorigenic and a promising therapeutic target, whereas GLS2 behaves more like a tumor suppressor factor despite some controversial results. GLS1 is overexpressed in various cancer cells, and this phenotype is associated with a higher disease stage and a poor prognosis. Mechanistically, the expression of GLS1 is regulated indirectly by Myc and mTORC1. Unlike GLS1, GLS2 suppresses the proliferation and migration of cancer cells.

The invention is based on the fact that, the selection of all or part of the group of genes disclosed above for the construction of the gene signature is optimal, meaning large enough to be robust against noise and small enough to be applied in a clinical setting. The gene signature allows identifying the presence of two molecular categories of GAC, (i) a first category named as the "inflamed class," that is based on the enrichment of gene-expression signatures defining tumor immunogenicity (i.e. antigen presentation) and anti-tumor immune activity (i.e. interferon signaling), and (ii) a second category that groups all the tumors that don't belong to the inflamed class.

In one preferred embodiment, the gene signature comprises all of the following: CD74, CD5, HLA-E, CD3E, ITGAL, CCL20, BLM, ADORA2A, RPL23 and GLS. In all of these embodiments, the gene signature may comprise one or more additional genes different from those disclosed in the above-numbered group of genes. This means that, the defined gene signature may comprise any further gene directed to determine the class of a GAC tumor, different from that of the cited genes.

As understood by a skilled person, when the gene signature consists of the above-mentioned genes, methods for performing an analysis may comprise measuring the expression of additional genes (e.g., for normalization) but only the gene signature is used to classify an individual.

The methods disclosed herein comprise determining the gene signature, more specifically the methods comprise determining the level of expression of the genes that form the signature in the tumor sample. Gene expression levels can be determined by measuring the level of nucleic acid or protein expression. The nucleic acid or protein is purified from the sample and gene expression is measured by nucleic acid or protein expression analysis. Preferably, the nucleic acid is mRNA, such as mRNA or pre-mRNA. Preferably, nucleic acid expression levels are determined. The level of nucleic acid expression may be determined by any method known in the art including RT-PCR, quantitative PCR, Northern blotting, gene sequencing, in particular RNA sequencing, and gene expression profiling techniques. Representative methods for sequencing-based gene expression analysis include Serial Analysis of Gene Expression (SAGE), and gene expression analysis by massively parallel signature sequencing (MPSS). As understood by a skilled person, the level of RNA expression determined may be detected directly or it may be determined indirectly, for example, by first generating cDNA and/or by amplifying the RNA/cDNA. In some embodiments, a gastric adenocarcinoma sample is obtained; RNA is extracted from the tissue sample; followed by reverse transcribing an RNA transcript of the genes of interest (e.g., biomarkers and housekeeping genes) to produce cDNAs of the RNA transcripts; and amplifying the cDNAs to produce amplicons from the cDNAs for determination of expression levels of the RNA transcripts. In some embodiments, gene expression may be determined by NanoString gene expression analysis. NanoString is a multiplexed method for detecting gene expression and provides a method for direct measurement of mRNAs without the use of transcription or amplification. NanoString and aspects thereof are described in Geiss et al., "Direct multiplexed measurement of gene expression with color- coded probe pairs" Nature Biotechnology 26, 317 - 325 (2008). The level of expression need not be an absolute value but may rather be a normalized expression value or a relative value. For example, the levels of expression can be normalized against housekeeping or reference gene expression. Such genes include ABCF1, ACTB, ALAS1, CLTC, G6PD, GAPDH, GUSB, HPRT1, LDHA, PGK1, POLR1B, POLR2A, RPL19, RPLPO, SDHA, TBP, and TUBB. Normalization is also useful when expression is determined based on microarray data. Normalization allows for correction for variation within microarrays and across samples so that data from different chips can be simultaneously analyzed. Additional methods of normalizing expression data are described in US20060136145.

Alternatively, it is also possible to determine the expression levels of the genes of interest by means of the determination of the expression levels of the proteins encoded by the genes comprising the gene signature, since if the expression of genes is increased, an increase of the amount of corresponding protein should occur. The determination of the expression levels of the different proteins can be carried out using any conventional method. By way of a non-limiting example, said determination can be carried out using antibodies with the capacity for binding specifically to the protein to be determined (or to fragments thereof containing the antigenic determinants) and subsequent quantification of the resulting antigen-antibody complexes. The antibodies that are going to be used in this type of assay can be, for example polyclonal sera, hybridoma supernatants or monoclonal antibodies, antibody fragments, Fv, Fab, Fab' and F(ab')2, scFv, diabodies, triabodies, tetrabodies and humanized antibodies. At the same time, the antibodies may or may not be labeled. Illustrative, but non-exclusive, examples of markers that can be used include radioactive isotopes, enzymes, fluorophores, chemiluminescent reagents, enzyme cofactors or substrates, enzyme inhibitors, particles, dyes, etc. There is a wide variety of well-known assays that can be used in the present invention, using non-labeled antibodies (primary antibody) and labeled antibodies (secondary antibodies); these techniques include Western-blot or immunoblot, ELISA (enzyme-linked immunosorbent assay), RIA (radioimmunoassay), competitive EIA (enzyme immunoassay), DAS-ELISA (double antibody sandwich ELISA), immunocytochemical and immunohistochemical techniques, techniques based on the use of biochips or protein microarrays including specific antibodies or assays based on the colloidal precipitation in formats such as reagent strips. Other forms of detecting and quantifying the protein include affinity chromatography techniques, ligand-binding assays, etc.

The methods described herein allows classifying an individual based on gene signatures. In some embodiments, the differential expression of one or more genes of the signature in a tumor sample indicates the class of the tumor, or rather, indicates the prognosis indicates the prognosis of the individual. As used herein, "differentially-expressed" means that the measured expression level in a tumor sample from a subject differs significantly from a reference. The reference may be a single value or a numerical range. It is within the purview of a skilled person to determine the appropriate reference value. In some embodiments, the reference value is a predetermined value. In some embodiments, the reference value is the average of the expression value in a particular patient class. In some embodiments, the reference value may be the average of the expression value in a class of patients that which do not suffer gastric carcinoma or which do not have a history of GAC. In some embodiments, the reference value may be the average of the expression value in a class of patients that have clinically confirmed GAC. A reference value may also be in the form of or derived from an equation. It is within the purview of one skilled in the art to determine whether the expression level in the patient differs "significantly" from a reference. In some embodiments, the gene signature from a tumor sample is compared to the reference expression signature to determine whether the gene signature from the tumor sample is sufficiently similar/different to the reference profile. Alternatively, the gene signature from an individual is compared to a plurality of reference expression signatures to select the reference expression profile that is most similar to the gene expression profile from an individual. Any method known in the art for comparing two or more data sets to detect similarity between them may be used to compare the gene signature from an individual to the reference expression profiles. As understood by a skilled person, training of a gene signature can be performed to favor sensitivity or specificity. Sensitivity refers to the proportion of actual positives that are correctly identified as such and high sensitivity is desired to avoid false negatives (e.g., patients classified as metastasis negative that are in fact positive).

In a preferred embodiment, the in vitro method for determining the class of a tumor from an individual with GAC, the gene signature comprises all of: CD74, CD5, HLA-E, CD3E, ITGAL, CCL20, BLM, ADORA2A, RPL23 and GLS genes.

In a preferred embodiment, the in vitro method for determining the class of a tumor from an individual with GAC, the tumor is classified as tumor of the inflamed class when the expression values of at least four genes of the first subgroup of genes consisting of group CD74, CD5, HLA-E, CD3E, and ITGAL, are overexpressed compared to a reference value; and when the expression level of at least four genes selected from a second subgroup of genes consisting of CCL20, BLM, ADORA2A, RPL23, and GLS, are infra-expressed compared to a reference value. The tumor is not classified as tumor of the inflamed class when the expression level of at least four genes from the first subgroup is not over-expressed compared to the reference value; and the expression level of at least four genes of the second subgroup are not infra-expressed compared to the reference value.

A second object of protection is constituted by an in vitro method for classifying an individual afflicted with GAC as in claim 5. The method is based on the classification of the tumor from said individual as GAC of the "inflamed class", as defined above. Specifically, the method comprises:
- determining the class of a tumor from an individual with GAC according to any one of claims 1 to 4;
wherein the individual is classified as having or not a gastric adenocarcinoma of the inflamed class based on the class of the tumor.

A third object of protection is constituted by an in vitro method predicting the prognosis of an individual with GAC as in claim 6. Specifically, the method comprises:
- determining the class of a tumor from an individual with GAC according to any one of claims 1 to 4; and
- determining the prognosis of said individual based on class of the tumor.

The disclosed gene signature allows classifying the prognosis of the individual. As used herein, prognosis refers to the prediction of a medical outcome and can be based on measures such as overall survival, recurrence free survival, relapse free survival and distant relapse free survival.

The individual may be thus classified as having a poor prognosis or a good prognosis.

A fourth object of protection is constituted by an in vitro method for defining a diagnostic work-up schedule and/or a treatment work-up schedule, and estimating the treatment response for an individual afflicted with gastric adenocarcinoma as in claim 7. Specifically, the method comprises, after determining the class of a tumor from the individual according to any one of claims 1 to 4:
- classifying the individual as having or not a gastric adenocarcinoma of the inflamed class; and
- determining the estimation of the response to an ICI treatment;
wherein there is an enhanced clinical benefit from ICI treatment if the tumor is classified as the inflamed class, or wherein there is a decreased clinical benefit from ICI treatment if said tumor is not classified as the inflamed class.

It has been shown that one of the potential clinical utilities of the "inflamed class" of GAC is the prediction of response to immunotherapies. The GAC tumors that don't belong to the inflamed class, on the other hand, displayed a worse clinical outcome in terms of time to recurrence and was enriched in TP53 mutations, a feature that has been also proposed to derive decreased clinical benefit from ICIs in GAC.

The term "estimating the treatment response", is used herein to refer to the likelihood that a patient will have a particular clinical outcome, whether positive or negative. The predictive methods of the present invention can be used clinically to make treatment decisions by choosing the most appropriate treatment modalities for any particular patient. The predictive methods of the present invention are valuable tools in predicting if a patient is likely to respond favorably to a treatment regimen, such as immunotherapy.

A fifth object of protection is constituted by a cancer treatment for use in treating an individual afflicted with GAC. Specifically, the method comprises:
- determining the class of a tumor from an individual with GAC according to any one of claims 1 to 4;
- classifying the individual as having GAC of the "inflamed class" based on the gene signature; and
- treating said individual by providing a cancer treatment to said individual, wherein said cancer treatment is immunotherapy treatment, preferably in combination with a treatment selected from the group consisting of radiotherapy, chemotherapy, target therapy, and any combination thereof.

In a preferred embodiment, the cancer treatment for use in treating an individual with GAC is an immunotherapy treatment selected from the group of cytokines, ICls, or oncolytic immunotherapy treatments.

A sixth object of protection is constituted by a kit as in claim 8, that comprises reagents adequate for determining the gene signature disclosed in the present invention, including any variation or alternative. Specifically, the kit comprises:
- reagents adequate for determining the expression level of the first subgroup consisting of CD74, CD5, HLA-E, CD3E, and ITGAL; and the second subgroup consisting of CCL20, BLM, ADORA2A, RPL23 and GLS; wherein the reagents are selected from the group consisting of primers, probes, aptamers and antibodies; and wherein the reagents comprise at least 10% of the total amount of reagents adequate for the determination of the expression levels of genes forming the kit.

In a more preferred embodiment, the kit of the invention comprises reagents adequate for the determination of the expression level of one or more housekeeping genes.

A seventh object of protection is constituted by the use of a kit for predicting the prognosis of an individual with GAC, as in claim 9. Specifically, the use of the kit comprises:
- predicting the prognosis of an individual afflicted with GAC; and
- selecting a suitable treatment for an individual afflicted with GAC or for an individual which is likely to benefit from immunotherapy treatment, preferably in combination with a treatment selected from the group consisting of radiotherapy, chemotherapy, target therapy, and any combination thereof.

An eight object of protection is constituted by a computer-implemented method for predicting the prognosis of an individual with GAC according to claim 6, or for selecting an adjuvant immunotherapy for an individual with GAC, as in claim 11. Specifically, the method comprises, after determining the class of a tumor from an individual with GAC according to any one of claims 1 to 4:
- analyzing the collected data by: comparing the expression levels of the genes with a reference value for each gene or; and
- providing the result of the analysis.

### EXAMPLES

### Example 1: Description of the study cohort

A study cohort of 82 patients allowed an integrative molecular analysis of GAC (Table 1). The study cohort was composed of a total of 82 GAC patients surgically resected between 2010 and 2020 and hat did not receive treatment with neoadjuvant prior to sample obtention. The study included patients with mostly early-stage disease (98% were AJCC I, II or III) with a variety of anatomical locations (8% proximal, 33% body and 59% distal) and histology (70% intestinal, 18% diffuse and 12% mixed) that were treated according to standard procedures (distal or total gastrectomy with lymphadenectomy followed by adjuvant chemotherapy and/or radiotherapy when indicated) and with a median overall survival of 72 months. Table 1 contains the clinical and pathological description of the study cohort.

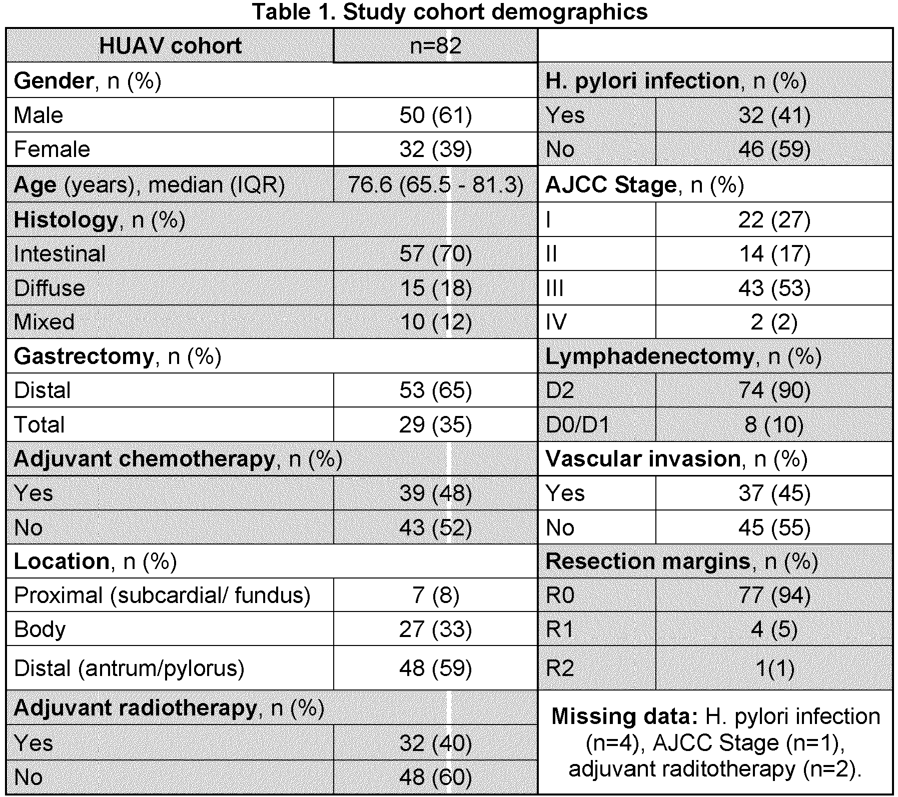

### Example 2: Studies conducted in the tumor samples

A total of 82 patients with GAC participated in the study, from which available frozen and formalin-fixed paraffin-embedded tumor samples were retrospectively collected at Arnau de Vilanova University Hospital. Frozen samples were macro-dissected to remove surrounding non-tumoral tissue and increase tumor purity. Clinical and pathological variables were collected from patients, including: demographics, risk factors, anatomical location, histology, AJCC pathological staging 8th edition, treatments received, and clinical outcomes in terms of recurrence and survival.

The study protocol was approved at center's Institutional Review Board. Patients included in the study provided written consent form. Samples collected were from the diagnostic surplus and stored at Biobank of the IRBLleida. The study was conducted in accordance with the Declaration of Helsinki.

Total RNA was isolated from frozen samples using the Maxwell RSC Simply RNA kit. RNA quantity was assessed on Qubit and RNA integrity was obtained using the TapeStation system. Tumors with >50% of purity and high RNA concentration (>20 ng/µl) were selected (N=57). Up to 5µl of RNA was used for gene expression profiling through the nCounter PanCancer IO 360, a curated panel including 770 genes across key immuno-oncology pathways and processes. Data importing, normalization, and sample calibration were conducted on the RCC files in nSolver Version 4.0. Files were imported with QC in the default setting. Next, positive control normalization followed the default setting. The CodeSet normalization was applied against 11 housekeeping genes with >150 counts and low mean standard deviation (%CV<60). The panel standard in each batch was selected in the CodeSet calibration step. One sample was filtered out due to mRNA positive normalization flag, ending up with 56 samples with normalized NanoString count data.

Cell type signature gene sets from MSigDB collections, representing 830 cluster marker genes for cell types identified in single-cell sequencing studies of human tissue, were evaluated using Gene Set Enrichment Analysis (GSEA) from GenePattern. On the other hand, EcoTyper was used to determine how cells organize into distinct cellular communities and establishing specific ecosystems across human solid tumors.

Gene signatures defining T cell dysfunction and exclusion as two primary mechanisms of tumor immune evasion were assessed according to the computational framework Tumor Immune Dysfunction and Exclusion (TIDE), which also infers the abundance of myeloid-derived suppressor cells (MDSC), cancer-associated fibroblasts (CAF), M2 tumor-associated macrophages (TAM M2) and predicted response to ICIs.

DNA was isolated from frozen samples with available remaining tissue (N=73) using the Maxwell RSC DNA kit. DNA quantity was assessed on Qubit. To assess the quality of DNA samples, purity, integrity and concentration was analyzed. To evaluate both the nuclear and mitochondrial genomes, KAPA HyperExome Probes (Roche) were used for whole exome enrichment in conjunction with KAPA HyperCap DS Human mtDNA Design of the KAPA HyperChoice Probes (Roche). Short-insert paired-end libraries for bigenomic capture were prepared using the KAPA HyperCap Workflow v3.0 (Roche). The libraries were prepared starting with 100ng of genomic DNA and using Illumina platform-compatible adapters with unique dual indexes and unique molecular identifiers (Integrated DNA Technologies). The captured libraries were then quality controlled for concentration and size using the Agilent Bioanalyzer DNA 7500 chip. The libraries were sequenced on NovaSeq 6000 (Illumina) with a read length of 2x151bp according to the manufacturer's protocol for dual indexing. The mean coverage was 273x. Image analysis, base calling, and quality scoring of the run were performed using the manufacturer's software Real Time Analysis (RTA v3.4.4), followed by generation of FASTQ sequence files.

After sequencing, reads were mapped to the human genome (hs37d5) using the BWA-mem with default parameters. Alignment files (BAM format) containing only properly paired, uniquely mapping reads were processed using Picard tools version 1.110 (http://broadinstitute.github.io/picard/) to add read groups and remove duplicates. The Genome Analysis Tool Kit was used for local indel realignment and base recalibration. Processed BAM files were used to perform somatic variant calling of single nucleotide variants and small insertions and deletions with Mutect2 (from GATK version 4.0.8.1). Since matched control samples were not available Mutect2 was run in tumor-only mode. To further discriminate somatic from germline variants two sets of variants were provided to Mutect2: (1) the aggregate variants from a "panel of normals" of 400 individuals, and (2) a set of human variants from gnomAD (https://gnomad.broadinstitute.org) as included in GATK.

The program FiltererdMutectCalls, from Mutect2, was used to filter out low quality single nucleotide variants and indels. Except for the "clustered events" filter, the default filters were used. To further exclude potential artefacts, variants were required to have at least 8 reads supporting the alternative allele and an alternative allele frequency greater than 0.05. The gene cancer analysis, tumor mutational burden and mutational profiles was computed using Maftools. The microsatellite instability analysis was performed with MSlsensor-pro.

Unstained 5µm FFPE sections (N=71) placed on Super Frost Plus slides were profiled using the GeoMx Digital Spatial Profiler (DSP) at different regions of interest (invasive margin, tumor center and between both regions) and compartments (tumoral cells and immune cells). Once the slides were deparaffined and subjected to antigen retrieval procedures, they were co-incubated with fluorescent-labeled visualization antibodies (panCK and CD45) together with 34 unique photocleavable oligonucleotide-labeled primary antibodies included in the Immune Cell Profiling Core (Beta-2-microglobulin, CD45, PD-1, CD11c, CD56, Fibronectin, PD-L1, CD20, CD68, GZMB, SMA, CD3, CD8, HLA-DR, CD4, CTLA4, Ki-67), the IO Drug Target Module (4-1BB, LAG3, ARG1, OX40L, B7-H3, STING, GITR, TIM-3, IDO1, VISTA) and the Immune Activation Status Module (CD80, CD25, ICOS, CD27, PD-L2, CD40, CD44).

Next, slides were loaded in a GeoMx DSP instrument, where they were scanned to produce a digital fluorescent image of the tissue that allowed selection of regions of interest by gastrointestinal pathologists. Oligos from the tumoral compartment (panCK+) and immune cell compartment (CD45+) were released upon exposure to UV light in a sequential manner. Photocleaved oligos were collected and dispensed into a 96-well plate where they were digitally counted in the nCounter system (NanoString). Resource Compiler (RCC) files from the 426 compartments were normalized to internal spike-in controls. One of them was excluded for further analysis due to low surface area and 26 due to high positive control normalization. Finally, counts were normalized based on the area of its compartment.

FFPE tissue blocks were sectioned at a thickness of 3µm, dried for 1h at 65° before pre-treatment procedure of deparaffinization, rehydration and epitope retrieval in the Pre-Treatment Module, PT-LINK (Agilent Technologies-DAKO, Santa Clara, United States) at 95°C for 20 min in 50x Tris/EDTA buffer, pH 9. Before staining the sections, endogenous peroxidase was blocked. After incubation with the primary antibody, the reaction was visualized with the EnVisionTM FLEX Detection Kit for p53, EnVisionTM FLEX Detection Kit +Linker (Mouse) for CD45, E-Cadherin, and MSH2 and EnVisionTM FLEX Detection Kit +Linker (Rabbit) for MLH1, MSH6 and PMS2. Immunostaining for PD-L1, HER2 and Claudin18 was performed according to manufacturer's instructions. Immunohistochemical results were evaluated by following uniform pre-established criteria. For MMR proteins (MLH1, MSH2, MSH6 and PMS2) loss of protein expression was defined as a complete absence of nuclear staining within the tumor. For HER2, a scoring 0, 1+, 2+, 3+ was used according to gastric cancer guidelines. For PD-L1 protein expression, Combined Positive Score (CPS) was obtained, which is the number of PD-L1 staining cells (tumor cells, lymphocytes, macrophages) divided by the total number of viable tumor cells, multiplied by 100. Finally, for Claudin18 a percentage of positivity was calculated considering moderate-high expression of tumoral cells. For p53 and E-Cadherin, immunexpression was graded semi-quantitatively by considering the percentage and intensity of the staining. A histological score was obtained from each sample, which ranged from 0 (no immunoreaction) to 300 (maximum immunoreactivity). Immunostaining localization, distribution and intensity were assessed by 3 independent gastrointestinal pathologists blinded to clinical and molecular features.

The detection of Eptein-Barr Virus in FFPE samples was performed automatically in the Benchmark GX apparatus following the manufacturer's instructions The fluorescein-labeled probe (INFORM EBER (Eptein-Barr Virus Early RNA) Probe) hybridized with the target sequence, followed by the streptavidin-AP (Alkaline Phosphatase) enzyme conjugate, which was added, and bound to the biotin present in the secondary antibody. Fluorescein-labeled probe was visualized with BCIP and nitroblue tetrazolium (NBT) as chromogen. It was counterstained with Red Stain II.

HER2 gene amplification was analyzed using the HER2 IQFISH pharmDx assay according to manufacturer's recommendations. This manual assay contains all key reagents required to complete a FISH procedure for sectioned FFPE specimens. Briefly, the specimen sections were exposed to heat pre-treatment and pepsin digestion. Next, denaturation step was performed for 10 min at 66°C followed by hybridization at 45°C using a Hybridizer. The hybridization was performed using the RTU FISH Probe Mix based on a combination of a Texas Red-labeled DNA probe (HER2) and a fluorescein-labeled PNA probe (CEN-17). Finally, the specimen sections were subjected to stringent wash at before dehydration and drying, and dried slides were subsequently mounted using Fluorescence Mounting Medium containing 4,6-diamidino-2-phenylindole (DAPI) and cover slipped. Gene amplification was determined from the ratio between the number of signals from the hybridization of the HER2 gene probe (red signals) and the number of signals from the hybridization of the CEN-17 reference chromosome 17 probe (green signals).

### Example 3: Characterization of genetic events in the study cohort

Tumors were evaluated for biomarkers used nowadays for the identification of patients suitable for targeted therapies for this disease. Up to 25% of the cases were mismatch repair-deficient (MMRd), whereas the rest (75%) were proficient MMR (MMRp). HER2 overexpression/amplification was present in 14% of the samples, being all from the pMMR class. PDL1 overexpression, on the other hand, was observed in 35% of tumors, with an enrichment of the dMMR class (p<0.001). In terms of CLDN18.2 overexpression, it was detected in 29% of tumors, being mutually exclusive with PDL1 overexpression (p=0.017). EBV was only identified in 4% of the cohort.

Whole-exome sequencing was applied in the cohort to identify somatic structural genetic alterations. Single nucleotide polymorphism was the more frequent variant type, originating mainly missense mutations. All MMRd tumors had a high MSI32 and a higher tumor mutation burden (TMB) than MMRp (median 1616 vs 471 variants per sample; p<0.001), as expected. In terms of frequently mutated driver genes described in GAC4, TP53 was the more prevalent (41%), with a long tail of less common genes, including the actionable46 PIK3CA (16%), KRAS (15%) and ERBB2 (14%). Overall, the distribution of genetic events in the cohort and within MMR classes was similar to the distribution found in previously published studies, and adequately portrays the known biological landscape of GAC.

### Example 4: Landscape of tumor infiltrating immune cells in GAC

Starting from the internal cohort, unstained 5µm FFPE sections (N=71) placed on Super Frost Plus slides were profiled using the GeoMx Digital Spatial Profiler (DSP) at different regions of interest (invasive margin, tumor center and between both regions) and compartments (tumoral cells and immune cells).

Once the slides were deparaffined and subjected to antigen retrieval procedures, they were co-incubated with fluorescent-labeled visualization antibodies (panCK and CD45) together with 34 unique photocleavable oligonucleotide-labeled primary antibodies included in the Immune Cell Profiling Core panel (Beta-2-microglobulin, CD45, PD-1, CD11c, CD56, Fibronectin, PD-L1, CD20, CD68, GZMB, SMA, CD3, CD8, HLA-DR, CD4, CTLA4, Ki-67), including its IO Drug Target Module (4-1BB, LAG3, ARG1, OX40L, B7-H3, STING, GITR, TIM-3, IDO1, VISTA) and its Immune Activation Status Module (CD80, CD25, ICOS, CD27, PD-L2, CD40, CD44).

Next, slides were loaded in a GeoMx DSP instrument, where they were scanned to produce a digital fluorescent image of the tissue that allowed selection of regions of interest by gastrointestinal pathologists. Oligos from the tumoral compartment (panCK+) and immune cell compartment (CD45+) were released upon exposure to UV light in a sequential manner. Photocleaved oligos were collected and dispensed into a 96-well plate where they were digitally counted in the nCounter system (NanoString).

RCC files from the 426 compartments were normalized to internal spike-in controls. One of them was excluded for further analysis due to low surface area and 26 due to high positive control normalization. Finally, counts were normalized based on the area of its compartment.

Immune infiltrates, assessed by CD45 immunostaining, were heterogeneous in the GAC cohort: from tumors displaying a high and uniform pattern of infiltration to tumors with a global low degree of infiltration. Of note, the Lauren histological classification qualitatively impacted the immune contexture of tumors, with the intestinal subtype producing a more arborescent infiltration surrounding tubular or glandular tumor formations, whereas the diffuse subtype producing a more isolated infiltration nearby to poorly cohesive cells and with a higher prevalence of lymphoid aggregates (p<0.001). Moreover, the region of the tumor determined the distribution of different types of immune cells present in GAC, being markers of T cells (p=0.023) more expressed at protein level in the invasive margin when compared to the tumor center.

Immune subpopulations infiltrating tumors were then estimated by gene-expression of immune metagenes and correlated with known histologies and biomarkers of GAC. Cytotoxic cells, the most powerful effectors in the anticancer immune response and the backbone of current successful ICls, were enriched in MMRd (p=0.008) and PDL1+ (p<0.001) tumors. However, the exhausted pattern of CD8 T cells was only observed in PDL1+ tumors (p=0.008). Conversely, NK cells were more present in the intestinal histology (p=0.007) as well as in MMRp (p=0.037) and HER2+ (p=0.007) subgroups. Diffuse histology was defined by a higher macrophage (p=0.041) and mast cell (p=0.002) infiltration. Finally, CLDN18.2 positivity determined lower amounts of CD8 T cells (p=0.049) and Tregs (p=0.033).

Several targets including checkpoint molecules were measured by multiplex protein profiling at the immune compartment of tumors. PD-L1 was overexpressed as expected in PDL1+ (p=0.001) and MMRd (p=0.005) tumors. ICOS, a T cell activating costimulatory immune checkpoint, was on the other hand downregulated in MMRd tumors (p=0.042). The enzyme IDO1, a proposed key factor of acquired immune tolerance, was particularly upregulated in PDL1+ tumors (p=0.001), but also in MMRd (p=0.012) and intestinal (p=0.020) subgroups. No significant associations were detected depending on HER2 status, whereas CLDN18.2 positivity was a related to higher expression of PD-1 (p=0.004), Tim-3 (p=0.036) and CTLA4 (p=0.002). These results reinforce the diversity of immunophenotypes present in GAC what warrants a rational use of immuno-oncology drugs.

### Example 5: Identification and immunophenotyping of the GAC Inflamed class

Total RNA was isolated from frozen samples using the Maxwell RSC Simply RNA kit. RNA quantity was assessed on Qubit and RNA integrity was obtained using the TapeStation system. Tumors with >50% of purity and high RNA concentration (>20 ng/µl) were selected (N=57). Up to 5µl of RNA was used for gene expression profiling through the nCounter PanCancer IO 360, a curated panel including 770 genes across key immuno-oncology pathways and processes. Samples were run on the nCounter prep station and scanner. Each batch contained a panel standard.

Data importing, normalization, and sample calibration were conducted on the RCC files in nSolver Version 4.0. Files were imported with QC in the default setting. Next, positive control normalization followed the default setting. The CodeSet normalization was applied against 11 housekeeping genes with >150 counts and low mean standard deviation (%CV<60). The panel standard in each batch was selected in the CodeSet calibration step. One sample was filtered out due to mRNA positive normalization flag, ending up with 56 samples with normalized NanoString count data.

Non-negative matrix factorization (NMF) from NMFConsensus module in GenePattern was employed to identify stable gene expression clusters (unsupervised clustering). NMF parameters: k = 1 to k = 5 clusters; number of clusterings to build consensus matrix = 20; number of iterations = 2000; error function = Euclidean. The preferred clustering result was determined using the observed cophenetic correlation, which measures the stability based on distances between clusters.

Unsupervised clustering by tumor microenvironment gene-expression (770 genes related with different features of the anti-tumoral immune response as well as the composition of the immune cells infiltrating the tumor) proposed the presence of essentially two molecular categories of GAC. The first category, including 52% of the tumors, was named as "the inflamed class" based on the enrichment of gene-expression signatures defining tumor immunogenicity (i.e. antigen presentation [p<0.001]) and anti-tumor immune activity (i.e. interferon signaling [p=0.005]). The second category, including the rest 48% of the tumors, was named as not belonging to "the inflamed class" during the experiments, based on the opposite gene-expression profile suggesting lower immunogenicity (presumably as a result of a more efficient DNA damage repair [p=0.020]) (Table 2). This second category groups all the tumors that don't belong to the inflamed class.

Table 2 contains gene signatures measuring biological variables crucial to the tumor-immune interaction (antigen availability, structural barriers to immune infiltration, inhibitory signaling, inhibitory metabolism, pro-immune signaling, killing of tumor cells, tumor receptiveness to immune signaling, tumor proliferation and apoptosis) up- or downregulated in the Inflamed class of GAC. Samples from the same group were represented with a normalized enrichment score.

**Table 2. Gene signatures analysis**

| | | CD45 (Inflamed class vs. Rest*) | | CD45 Margin (Inflamed class vs. Rest*) | | CD45 Center (Inflamed class vs. Rest*) | |
|---|---|---|---|---|---|---|---|
| | Target | Counts | P value | Counts | P value | Counts | P value |
| Leucocytes | CD45 | 0,4 | 0,2 | -0,1 | 0,3 | 1,0 | 0,018* |
| B cells | CD20 | 0,5 | 0,2 | 0,4 | 0,9 | 0,9 | 0,7 |
| T cells | CD3 | -0,1 | 0,8 | -0,2 | 0,6 | 0,2 | 0,4 |
| Cytotoxic T | CD8 | 0,0 | 1,0 | -0,1 | 0,7 | 0,4 | 0,2 |
| cells | | | | | | | |
| Helper T cells | CD4 | 0,1 | 0,8 | -0,1 | 0,1 | 0,5 | 0,4 |
| Tregs | CD25 | 0,0 | 0,9 | -0,1 | 0,3 | 0,5 | 0,1 |
| NK cells | CD56 | 0,5 | 0,1 | 0,4 | 0,8 | 0,8 | 0,1 |
| Dendritic cells | CD11c | 0,5 | 0,1 | 0,1 | 0,7 | 1,0 | 0,1 |
| Macrophages | CD68 | 0,4 | 0,2 | 0,3 | 0,4 | 0,6 | 0,1 |
| Antigen presentation | HLA-DR | 0,5 | 0,1 | 0,4 | 0,8 | 1,1 | 0,020* |
| | B2M | 0,4 | 0,2 | 0,3 | 0,6 | 0,8 | 0,3 |
| T cell regulation | ICOS | -0,1 | 0,5 | -0,6 | 0,038* | 0,3 | 0,2 |
| | CD44 | 0,6 | 0,1 | 0,0 | 1,0 | 1,4 | 0,003* |
| | GITR | 0,4 | 0,2 | 0,2 | 1,0 | 0,6 | 0,4 |
| | 4-1BB | 0,0 | 1,0 | -0,3 | 0,044* | 0,6 | 0,2 |
| | CD27 | 0,5 | 0,044* | 0,4 | 0,6 | 0,8 | 0,017* |
| Myeloid cell regulation | CD80 | 0,0 | 1,0 | 0,0 | 1,0 | 0,6 | 0,1 |
| | CD40 | 0,0 | 0,9 | -0,1 | 0,9 | 0,2 | 0,7 |
| | ARG1 | 0,2 | 0,4 | 0,1 | 0,9 | 0,6 | 0,6 |
| | OX40L | 0,2 | 0,3 | 0,2 | 0,7 | 0,4 | 0,2 |
| | IDO1 | 0,4 | 0,3 | 0,0 | 0,8 | 1,1 | 0,2 |
| Checkpoints | PD-1 | 0,3 | 0,3 | 0,2 | 0,6 | 0,6 | 0,5 |
| | LAG3 | 0,3 | 0,1 | 0,0 | 0,8 | 0,8 | 0,017* |
| | Tim-3 | 0,5 | 0,2 | 0,4 | 0,2 | 0,9 | 0,2 |
| | CTLA4 | 0,1 | 0,7 | 0,0 | 0,7 | 0,3 | 0,3 |
| | PD-L1 | 0,5 | 0,1 | 0,3 | 0,6 | 0,7 | 0,2 |
| | PD-L2 | 0,2 | 0,2 | 0,1 | 0,6 | 0,5 | 0,2 |
| | B7-H3 | -0,2 | 0,5 | -0,3 | 0,2 | 0,2 | 0,8 |
| | VISTA | 0,2 | 0,3 | -0,1 | 0,6 | 0,6 | 0,1 |
| Cytotoxicity | STING | 0,9 | 0,1 | -0,2 | 0,1 | 1,9 | 0,002* |
| | GZMB | 0,4 | 0,1 | 0,4 | 0,4 | 0,5 | 0,3 |

| | | | | | | | |
|---|---|---|---|---|---|---|---|
| **Rest: tumors that don't belong to the inflamed class.* | | | | | | | |

After interrogating 830 gene sets representing human cell types, the pattern of expression of gastric immune cells was the more similar to the one depicted by the Inflamed class. Indeed, gene-expression deconvolution inferred increased amounts of a variety of immune cells in this tumor class when compared to tumors that don't belong to the inflamed class, particularly in terms of CD8 T lymphocytes with an exhaustion pattern (p<0.001). When analyzing ten previously proposed ecosystems across human solid tumors, we identified that the predominant multicellular community of the Inflamed class of GAC was proinflammatory, leucocyte rich and characterized by higher immunoreactivity. In terms of spatial protein profiling, tumors from the Inflamed class were characterized by increased expression by immune cells of the MHC II molecule HLA-DR (p=0.020) as well as T cell regulation and cytotoxicity markers such as STING (p=0.002), exclusively in the tumor center and not in the tumor margin (Table 3).

Table 3 describes the spatial protein profiling of anti-tumoral immune response markers in the immune compartment (CD45+) of the Inflamed class of GAC according to different regions of interest (invasive margin and tumor center).

**Table 3. Anti-tumoral immune response in the immune compartment**

| | Inflamed | Rest* | P value |
|---|---|---|---|
| Antigen Presentation | 0,49 | -0,53 | <0.001* |
| NF-kappaB Signaling | 0,44 | -0,48 | <0.001* |
| Lymphoid Compartment | 0,44 | -0,47 | <0.001* |
| Immune Cell Adhesion and Migration | 0,43 | -0,46 | 0,00051* |
| Costimulatory Signaling | 0,41 | -0,45 | 0,00083* |
| Apoptosis | 0,40 | -0,43 | 0,0015* |
| JAK-STAT Signaling | 0,39 | -0,42 | 0,0019* |
| Interferon Signaling | 0,35 | -0,38 | 0,0051* |
| Autophagy | 0,33 | -0,36 | 0,0091 * |
| Cytotoxicity | 0,29 | -0,31 | 0,023* |
| Cytokine and Chemokine Signaling | 0,27 | -0,29 | 0,033* |
| PI3K-Akt | 0,27 | -0,29 | 0,034* |
| Matrix Remodeling and Metastasis | 0,23 | -0,25 | 0,068 |
| MAPK | 0,23 | -0,25 | 0,074 |
| Hypoxia | 0,21 | -0,22 | 0,11 |
| Hedgehog Signaling | 0,20 | -0,22 | 0,12 |
| Notch Signaling | 0,15 | -0,17 | 0,23 |
| Myeloid Compartment | 0,15 | -0,16 | 0,24 |
| Metabolic Stress | 0,15 | -0,16 | 0,24 |
| TGF-beta Signaling | 0,14 | -0,16 | 0,27 |
| Wnt Signaling | 0,14 | -0,15 | 0,27 |
| Epigenetic Regulation | 0,10 | -0,10 | 0,45 |
| Angiogenesis | 0,060 | -0,064 | 0,65 |
| Cell Proliferation | -0,20 | 0,21 | 0,12 |
| DNA Damage Repair | -0,30 | 0,32 | 0,020* |

| | | | |
|---|---|---|---|
| **Rest: tumors that don't belong to the inflamed class.* | | | |

### Example 6: Clinical and molecular characterization of the GAC Inflamed class

Regarding clinical, pathological and molecular correlates, it was noticed that, in the internal cohort, all tumors with diffuse histology were classified as Inflamed (p=0.112). On the other hand, no association was detected between the Inflamed class and the presence of MMRd (p=0.562) or PDL1 overexpression (p=0.425). Despite not significant, a higher prevalence of mutations in PIK3CA (30% vs 10%, p=0.152) and ARID1A (41% vs 24%, p=0.355) was observed in the Inflamed class. TP53 mutations, on the contrary, were more common in GACs that don't belong to the inflamed class (67% vs 52%, p=0.382). Patients with tumors from the Inflamed class had a better clinical outcome in terms of time to recurrence in comparison to the rest of patients (p=0.030). In summary, a novel Inflamed class of GAC that captures a unique tumor microenvironment fingerprint has been identified.

Noticeable, the Inflamed class of GAC was associated to the induction of a T cell dysfunction (p<0.001) and with a predicted increased clinical response to ICls (p=0.039), whereas the GACs that don't belong to the inflamed class were linked to the exclusion of T cell infiltration (p<0.001) mediated mainly by MDSC (p<0.001). In this regard, targetable co-inhibitory receptors of the tumor-immune synapse such are PD1 (p=0.039), CTLA4 (p=0.048), LAG3 (p=0.013) or TIGIT (p=0.002) were overexpressed in the Inflamed class at RNA level. On the other hand, the also targetable VEGFA ligand was overexpressed in tumors that don't belong to the inflamed class (p=0.021).

### Example 7: Design, validation and clinical utility of the GAC Inflamed classifier gene signature

The Weighted Voting classification with Leave-One-Out Cross-Validation algorithm from GenePattern was used to select a weighted linear combination of relevant genes obtained in the training set to provide a classification scheme for new samples. The selection of classifier input features (marker genes) was accomplished by computing a T-Test. The model was tested in the leave-one-out cross-validation mode by iteratively leaving one sample out and training a model on the remaining data and testing on the left-out sample. The selection of 10 genes for the construction of the gene signature is large enough to be robust against noise and small enough to be applied in a clinical setting. The resulting gene signature was then used to validate the above-described Inflamed class of GAC.

The signature was able to identify GAC samples belonging to this class in the TCGA (N=265) and ACRG (N=300) external cohorts with a similar prevalence (46% and 54%, respectively) compared to the discovery HUAV dataset. The same enrichment of the Inflamed class of GAC in gene signatures defining tumor immunogenicity (p<0.001), anti-tumor immune activity (p<0.001) and high tumor-infiltrating lymphocytes with an exhaustion pattern (p<0.001) was validated in both external cohorts. Likewise, previously mentioned pathological and molecular associations were statistically confirmed for the Inflamed class of GAC when increasing sample size: First, the increased proportion of diffuse histology (p<0.001); second, the enrichment of PIK3CA (p<0.001) and ARID1A (p<0.001) mutations; and third, the lower proportion of TP53 mutations (p<0.001). The better prognosis in terms of time to recurrence of the Inflamed class of GAC could be proved in the ACRG cohort (p=0.046). The median follow-up for recurrence of only 12.75 months in the TCGA cohort prevented further validation.

The predictive capability of response to immunotherapies of the Inflamed class of GAC in human cohorts was calculated. For advanced GAC, one Asian cohort had available transcriptome data from 45 patients treated with pembrolizumab (PD-1 inhibitor) in second/third-line treatments. The Inflamed class of GAC was detected in up to 47% of the tumors of this cohort which represents a similar performance of the gene signature than in early clinical stages of GAC. The ORR was higher in patients from the Inflamed class when compared to patients with tumors that don't belong to the inflamed class (33% vs 21%).

Additional cohorts testing ICIs (PD-1, PD-L1 or CTLA4 inhibitors) or adoptive cellular therapies for other cancer types (melanoma, renal cell carcinoma and urothelial carcinoma) were used to assess the cross-validity of the biomarker.

Overall, after analyzing clinical responses to 994 patients, the average area under the receiver operating characteristic curve (AUC) for the Inflamed class of GAC was 0.63, outperforming other gene-expression signatures such as the T-cell inflamed signature44 (AUC 0.62) or the Tumor Immune Dysfunction and Exclusion score (AUC=0.57).

## Claims

1. An *in vitro* method for determining the class of a tumor from an individual with gastric adenocarcinoma, the method comprising:
- determining the level of gene expression of a gene signature in a tumor sample from said individual, wherein the gene signature comprises:
- a first subgroup of five genes consisting of CD74, CD5, HLA-E, CD3E, and ITGAL; and
- a second subgroup of five genes consisting of CCL20, BLM, ADORA2A, RPL23, and GLS;
wherein the tumor is classified as being or not of the inflamed class.

2. The *in vitro* method of claim 1, wherein the gene signature consists of the genes from the two subgroups: CD74, CD5, HLA-E, CD3E, ITGAL, CCL20, BLM, ADORA2A, RPL23 and GLS.

3. The *in vitro* method according to any one of claims 1 or 2, wherein:
- the tumor is classified as tumor of the inflamed class when the expression level of the five genes from the first subgroup are over-expressed compared to a reference value; and when the expression level of the five genes of the second subgroup are infra-expressed compared to a reference value;
- and the tumor is not classified as tumor of the inflamed class when the expression level of the five genes from the first subgroup are not over-expressed compared to the reference value; and the expression level of the five genes of the second subgroup are not infra-expressed compared to the reference value.

4. An *in vitro* method for classifying an individual afflicted with gastric adenocarcinoma, comprising:
- determining the class of a tumor from the individual according to any one of claims 1 to 3;
wherein the individual is classified as having or not a gastric adenocarcinoma of the inflamed class based on the class of the tumor.

5. An *in vitro* method for predicting the prognosis of an individual afflicted with gastric adenocarcinoma, comprising:
- determining the class of a tumor from the individual according to any one of claims 1 to 3; and
- determining the prognosis of said individual based on the class of the tumor.

6. An *in vitro* method for defining a treatment work-up schedule and estimating a treatment response to an Immune Checkpoint Inhibitor (ICI) for an individual afflicted with gastric adenocarcinoma, comprising:
- classifying the individual as having or not a gastric adenocarcinoma of the inflamed class by means of the method defined in claim 4; and
- determining the estimation of the response to an ICI treatment;
wherein an ICI treatment is scheduled for the individual if the tumor is classified as the inflamed class.

7. A kit comprising reagents adequate for determining the expression level of the group of genes consisting of CD74, CD5, HLA-E, CD3E, ITGAL, CCL20, BLM, ADORA2A, RPL23 and GLS; wherein the reagents are selected from the group consisting of primers, probes, aptamers and antibodies.

8. Use of a kit according to claim 7 for:
- determining the class of a tumor from an individual with gastric adenocarcinoma as being or not of the inflamed class;
- classifying an individual with gastric adenocarcinoma as having or not a gastric adenocarcinoma of the inflamed class;
- predicting the prognosis of an individual afflicted with gastric adenocarcinoma, or
- selecting an ICI treatment for
- an individual afflicted with gastric adenocarcinoma of the inflamed class, or
- an individual which is likely to benefit from ICI treatment.

9. Use of a kit according to claim 8, wherein the ICI treatment is combined with a treatment selected from the group consisting of radiotherapy, chemotherapy, target therapy, and any combination thereof.

10. A computer-implemented method for predicting the prognosis of an individual afflicted with gastric adenocarcinoma, and/or for defining a treatment work-up schedule for the individual, the method comprising analyzing the data collected through the *in vitro* method disclosed in any one of claim 1 or 2 by comparing the expression level of the genes with a reference level for each gene; and providing the predicting the prognosis, in such a way that:
- the tumor is classified as tumor of the inflamed class when the expression level of the five genes from the first subgroup are over-expressed compared to a reference value; and when the expression level of the five genes of the second subgroup are infra-expressed compared to a reference value;
- and the tumor is not classified as tumor of the inflamed class when the expression level of the five genes from the first subgroup are not over-expressed compared to the reference value; and the expression level of the five genes of the second subgroup are not infra-expressed compared to the reference value.

## Patentansprüche

1. In-vitro-Verfahren zum Bestimmen der Klasse eines Tumors bei einem Individuum mit einem Magenadenokarzinom, wobei das Verfahren Folgendes umfasst:
- Bestimmen des Niveaus der Genexpression einer Gensignatur in einer Tumorprobe von dem Individuum, wobei die Gensignatur Folgendes umfasst:
- eine erste Untergruppe von fünf Genen, die aus CD74, CD5, HLA-E, CD3E und ITGAL besteht; und
- eine zweite Untergruppe von fünf Genen, die aus CCL20, BLM, ADORA2A, RPL23 und GLS besteht;
wobei der Tumor als der entzündlichen Klasse zugehörend oder nicht zugehörend klassifiziert wird.

2. In-vitro-Verfahren nach Anspruch 1, wobei die Gensignatur aus den Genen aus den folgenden zwei Untergruppen besteht: CD74, CD5, HLA-E, CD3E, ITGAL, CCL20, BLM, ADORA2A, RPL23 und GLS.

3. In-vitro-Verfahren nach einem der Ansprüche 1 oder 2, wobei:
- der Tumor als ein der entzündlichen Klasse zugehörender Tumor klassifiziert wird, wenn das Expressionsniveau der fünf Gene aus der ersten Untergruppe im Vergleich zu einem Referenzwert überexprimiert ist; und wenn das Expressionsniveau der fünf Gene der zweiten Untergruppe im Vergleich zu einem Referenzwert unterexprimiert ist;
- und der Tumor nicht als ein der entzündlichen Klasse zugehörender Tumor klassifiziert wird, wenn das Expressionsniveau der fünf Gene aus der ersten Untergruppe im Vergleich zu dem Referenzwert nicht überexprimiert ist; und das Expressionsniveau der fünf Gene der zweiten Untergruppe im Vergleich zu dem Referenzwert nicht unterexprimiert ist.

4. In-vitro-Verfahren zum Klassifizieren eines Individuums, das an einem Magenadenokarzinom leidet, umfassend:
- Bestimmen der Klasse eines Tumors bei dem Individuum nach einem der Ansprüche 1 bis 3;
wobei das Individuum als Träger oder Nicht-Träger eines der entzündlichen Klasse zugehörenden Magenadenokarzinoms basierend auf der Klasse des Tumors klassifiziert wird.

5. In-vitro-Verfahren zum Vorhersagen der Prognose eines Individuums, das an einem Magenadenokarzinom leidet, umfassend:
- Bestimmen der Klasse eines Tumors bei dem Individuum nach einem der Ansprüche 1 bis 3; und
- Bestimmen der Prognose des Individuums basierend auf der Klasse des Tumors.

6. *In-vitro*-Verfahren zum Definieren eines Behandlungsplans und Abschätzen des Ansprechens auf eine mit einem Immun-Checkpoint-Inhibitor (ICI) durchgeführten Behandlung bei einem Individuum, das an einem Magenadenokarzinom leidet, umfassend:
- Klassifizieren des Individuums als Träger oder Nicht-Träger eines der entzündlichen Klasse zugehörenden Magenadenokarzinoms mittels des in Anspruch 4 definierten Verfahrens; und
- Bestimmen der Abschätzung des Ansprechens auf eine ICI-Behandlung;
wobei eine ICI-Behandlung für das Individuum geplant ist, wenn der Tumor als der entzündlichen Klasse zugehörend klassifiziert wird.

7. Kit, umfassend Reagenzien, die zum Bestimmen des Expressionsniveaus der Gruppe von Genen, die aus CD74, CD5, HLA-E, CD3E, ITGAL, CCL20, BLM, ADORA2A, RPL23 und GLS besteht, geeignet sind; wobei die Reagenzien aus der Gruppe, die aus Primern, Sonden, Aptameren und Antikörpern besteht, ausgewählt sind.

8. Verwendung eines Kits nach Anspruch 7 zum:
- Bestimmen der Klasse eines Tumors bei einem Individuum mit einem der entzündlichen Klasse zugehörenden oder nicht zugehörenden Magenadenokarzinom;
- Klassifizieren eines Individuums mit einem Magenadenokarzinom als Träger oder Nicht-Träger eines der entzündlichen Klasse zugehörenden Magenadenokarzinoms;
- Vorhersagen der Prognose eines Individuums, das an einem Magenadenokarzinom leidet, oder
- Auswählen einer ICI-Behandlung für
- ein Individuum, das an einem der entzündlichen Klasse zugehörenden Magenadenokarzinom leidet, oder
- ein Individuum, das wahrscheinlich von einer ICI-Behandlung profitieren wird.

9. Verwendung eines Kits nach Anspruch 8,
wobei die ICI-Behandlung mit einer Behandlung, die aus der aus Strahlentherapie, Chemotherapie, zielgerichteter Therapie und jeder Kombination davon bestehenden Gruppe ausgewählt ist, kombiniert wird.

10. Computerimplementiertes Verfahren zum Vorhersagen der Prognose eines Individuums, das an einem Magenadenokarzinom leidet, und/oder zum Definieren eines Behandlungsplans für das Individuum, wobei das Verfahren Folgendes umfasst: Analysieren der durch das in Anspruch 1 oder 2 offengelegte *In-vitro-*Verfahren gesammelten Daten durch Vergleichen des Expressionsniveaus der Gene mit einem Referenzniveau für jedes Gen; und Bereitstellen des Vorhersagens der Prognose auf eine Weise, dass:
- der Tumor als ein der entzündlichen Klasse zugehörender Tumor klassifiziert wird, wenn das Expressionsniveau der fünf Gene aus der ersten Untergruppe im Vergleich zu einem Referenzwert überexprimiert ist; und wenn das Expressionsniveau der fünf Gene der zweiten Untergruppe im Vergleich zu einem Referenzwert unterexprimiert ist;
- und der Tumor nicht als ein der entzündlichen Klasse zugehörender Tumor klassifiziert wird, wenn das Expressionsniveau der fünf Gene aus der ersten Untergruppe im Vergleich zu dem Referenzwert nicht überexprimiert ist; und das Expressionsniveau der fünf Gene der zweiten Untergruppe im Vergleich zu dem Referenzwert nicht unterexprimiert ist.

## Revendications

1. Procédé *in vitro* pour déterminer la classe d'une tumeur provenant d'un individu présentant un adénocarcinome gastrique, le procédé comprenant :
- la détermination du niveau d'expression génique d'une signature génique dans un échantillon de tumeur provenant dudit individu, dans lequel la signature génique comprend :
- un premier sous-groupe de cinq gènes constitué de CD74, CD5, HLA-E, CD3E et ITGAL ; et
- un second sous-groupe de cinq gènes constitué de CCL20, BLM, ADORA2A, RPL23 et GLS ;
dans lequel la tumeur est classée comme étant ou n'étant pas de la classe inflammatoire.

2. Procédé *in vitro* selon la revendication 1, dans lequel la signature génique est constituée des gènes provenant des deux sous-groupes : CD74, CD5, HLA-E, CD3E, ITGAL, CCL20, BLM, ADORA2A, RPL23 et GLS.

3. Procédé *in vitro* selon l'une quelconque des revendications 1 ou 2, dans lequel :
- la tumeur est classée comme tumeur de la classe inflammatoire lorsque le niveau d'expression des cinq gènes provenant du premier sous-groupe est surexprimé par rapport à une valeur de référence ; et lorsque le niveau d'expression des cinq gènes du second sous-groupe est sous-exprimé par rapport à une valeur de référence ;
- et la tumeur n'est pas classée comme tumeur de la classe inflammatoire lorsque le niveau d'expression des cinq gènes provenant du premier sous-groupe n'est pas surexprimé par rapport à la valeur de référence ; et le niveau d'expression des cinq gènes du second sous-groupe n'est pas sous-exprimé par rapport à la valeur de référence.

4. Procédé *in vitro* pour classer un individu souffrant d'un adénocarcinome gastrique, comprenant :
- la détermination de la classe d'une tumeur provenant de l'individu selon l'une quelconque des revendications 1 à 3 ; dans lequel l'individu est classé comme ayant ou n'ayant pas un adénocarcinome gastrique de la classe inflammatoire sur la base de la classe de la tumeur.

5. Procédé *in vitro* pour prédire le pronostic d'un individu souffrant d'un adénocarcinome gastrique, comprenant :
- la détermination de la classe d'une tumeur provenant de l'individu selon l'une quelconque des revendications 1 à 3 ; et
- la détermination du pronostic dudit individu sur la base de la classe de la tumeur.

6. Procédé *in vitro* pour définir un calendrier de protocole de traitement et estimer une réponse au traitement à un inhibiteur de point de contrôle immunitaire (ICI) pour un individu souffrant d'un adénocarcinome gastrique, comprenant :
- la classification de l'individu comme ayant ou n'ayant pas un adénocarcinome gastrique de la classe inflammatoire au moyen du procédé défini dans la revendication 4 ; et
- la détermination de l'estimation de la réponse à un traitement par ICI ;
dans lequel un traitement par ICI est programmé pour l'individu si la tumeur est classée comme étant de la classe inflammatoire.

7. Kit comprenant des réactifs adéquats pour déterminer le niveau d'expression du groupe de gènes constitué de CD74, CD5, HLA-E, CD3E, ITGAL, CCL20, BLM, ADORA2A, RPL23 et GLS ; dans lequel les réactifs sont choisis dans le groupe constitué par des amorces, des sondes, des aptamères et des anticorps.

8. Utilisation d'un kit selon la revendication 7 pour :
- la détermination de la classe d'une tumeur provenant d'un individu présentant un adénocarcinome gastrique comme étant ou n'étant pas de la classe inflammatoire ;
- la classification d'un individu présentant un adénocarcinome gastrique comme ayant ou n'ayant pas un adénocarcinome gastrique de la classe inflammatoire ;
- la prédiction du pronostic d'un individu souffrant d'un adénocarcinome gastrique, ou
- la sélection d'un traitement par ICI pour
- un individu souffrant d'un adénocarcinome gastrique de la classe inflammatoire, ou
- un individu qui est susceptible de bénéficier d'un traitement par ICI.

9. Utilisation d'un kit selon la revendication 8, dans laquelle le traitement par ICI est combiné avec un traitement choisi dans le groupe constitué par la radiothérapie, la chimiothérapie, la thérapie ciblée, et toute combinaison de celles-ci.

10. Procédé mis en œuvre par ordinateur pour prédire le pronostic d'un individu souffrant d'un adénocarcinome gastrique, et/ou pour définir un calendrier de protocole de traitement pour l'individu, le procédé comprenant l'analyse des données collectées par l'intermédiaire du procédé in vitro divulgué dans l'une quelconque des revendications 1 ou 2 en comparant le niveau d'expression des gènes avec un niveau de référence pour chaque gène ; et la fourniture de la prédiction du pronostic, de telle manière que :
- la tumeur est classée comme tumeur de la classe inflammatoire lorsque le niveau d'expression des cinq gènes provenant du premier sous-groupe est surexprimé par rapport à une valeur de référence ; et lorsque le niveau d'expression des cinq gènes du second sous-groupe est sous-exprimé par rapport à une valeur de référence ;
- et la tumeur n'est pas classée comme tumeur de la classe inflammatoire lorsque le niveau d'expression des cinq gènes provenant du premier sous-groupe n'est pas surexprimé par rapport à la valeur de référence ; et le niveau d'expression des cinq gènes du second sous-groupe n'est pas sous-exprimé par rapport à la valeur de référence.
